# EUROPEAN PATENT APPLICATION

(11) **EP 2 783 637 A1**
(43) Date of publication of application: **01.10.2014**
(21) Application number: 12851434.6
(22) Date of filing: 20.11.2012
(51) Int. Cl.: A61B 17/06

(54) **GRIPPER, NEEDLE HOLDER, AND ATTACHMENT**

(30) Priority: 25.11.2011 JP 2011257950
(71) Applicant: Senko Medical Instrument Mfg. Co., Ltd., Tokyo 113-0033 (JP)
(72) Inventor: MOTOMURA, Noboru, Tokyo 112-0003 (JP)
(74) Representative: Beck Greener
(86) International application number: PCT/JP2012/080089
(87) International publication number: WO 2013/077330

(57) **Abstract**

Provided is a ripper capable of efficient gripping in a narrow space. A gripper is provided with a first arm (10L) and a second arm (10R) which can open and close about a supporting pin (10c) and configured so that opposing surfaces (12L,12R) formed by opening and closing the first and second arms (10L, 10R) forming the holding portion (12). The gripper is characterized in that a pair of clipping members (16L, 16R) is connected with the holding portion (12) of the first arm (10L) and the second arm (10R) so that the pair of clipping members (16L, 16R) can rotate about support points (16C) relative to the holding portion (12), and in that a rotation manipulation portion (17) is connected with the clipping members (16L, 16R) at a position offset from the support points (16c) and applies a displacement to the position to which the rotation manipulation portion (17) is connected.

## Description

### Technical Field

The present invention relates to a gripper providing efficient gripping-manipulation in a narrow space and the like, and a needle holder providing efficient handling of a needle and efficient suture in a narrow space and the like, and an attachment for configuring the gripper and the needle holder.

### Background Art

As well known, during a surgery, to a living body, grip, perforation, suture and the like are required in various kinds of environment conditions, particularly a narrow space. Therefore, a gripper, including particularly foroeps and a needle holder and the like, is used (for example, see the patent literature 1 and the non-patent literature 1). For example, when the suturing-procedure is performed with a needle holder, it is general to handle the suture needle in a direction orthogonal to the longitudinal direction of the needle holder (hereinafter, sometimes referred to as the lateral direction)

On the other hand, recently, in a field of surgery, a minimally invasive surgery requiring a scopic surgery has been increasing. In such a minimally invasive surgery, efficient gripping-manipulation and efficient suturing-procedure are required in a narrow view. In order to perform efficiently the minimally invasive surgery to a narrow space as an object in the narrow view, it is necessary to perform proper procedure felicitous to the state of the surgical field, for example, it is necessary to precisely perform the procedures such as grip and perforation of the living body, and handle the suture needle in a longitudinal direction of the needle holder (hereinafter, sometimes referred to as the vertical-tangent direction).

### Citation List

### Patent Literature

PTL1: JP-A- H08-038492

### Non Patent Literature

NPL1: http://ja.wikipedia.org/wiki/%E6%8C%81%E9%87%9D%E5%99%A8

### Summary of Invention

### Technical Problem

However, it is not easy to provide: flexible grip in the narrow space by the gripper, such as posture change of an object, and re-hold of the object; and handling the needle with respect to the vertical-tangent direction by the needle holder. Therefore, there is a strong demand for technology to provide: flexible gripping-manipulation of the object in a narrow space; and handling of the needle in the vertical-tangent direction, preferably, in the direction intersecting the lateral direction in accordance with the state of suturing position.

The present invention is made in consideration of the above situations, and aims for providing a gripper enabling efficient grip in a narrow space, a needle holder enabling efficient handling of a needle and efficient suture in a narrow space, and an attachment for configuring the gripper and the needle holder.

### Solution to Problem

In order to solve the above problems, the present invention provides the following means.

The invention according to claim 1, is a gripper for gripping an object, comprising: a gripper main body which has a manipulation portion and a holding portion, where the holding portion opens and closes by manipulation to the manipulation portion, and the holding portion closes to hold the object; and a posture control portion which controls a posture of the object held by the holding portion, wherein the posture control portion comprises: a clipping portion being provided to the holding portion, which clips the object and changes a posture of the clipping portion relative to the holding portion in a state of clipping the object; and a posture manipulation portion being connected with the clipping portion, which manipulates the posture of the clipping portion.

The invention according to claim 5 is an attachment being applied to a gripper main body having a manipulation portion and a holding portion, the holding portion being allowed to open and close by manipulation to the manipulation portion, and closing to hold an object, wherein the attachment has a posture control portion which controls a posture of the object held by the holding portion, and the posture control portion has: a clipping portion which is provided to the holding portion, and which clips the object and is allowed to change a posture of the clipping portion relative to the holding portion while clipping the object; and a posture manipulation portion which is connected with the clipping portion, and operates the posture of the clipping portion.

According to the gripper and the attachment of the present invention, since it is possible to change the posture of the object which is clipped by the clipping portion (the object is a suture needle in a case of the needle holder. The same applies to the following.), it is possible to change efficiently the posture of the object in a narrow space.

As a result of that, for example, it is possible to perform correctly procedures such as clip of the living body and perforation, and to handle safely and efficiently a needle, the posture of which intersects a lateral direction (a direction orthogonal to a longitudinal direction of the needle holder) such as a vertical-tangent direction.

According to the attachment of the present invention, it is possible to configure the gripper easily and efficiently by adding the attachment to a conventional gripper device (corresponding to the gripper main body of the present invention), which is a forceps type, a Castro Viejo needle holder type, a pincette, or straight grasping forceps for abdominal cavity surgery. As a result of that, it is possible to change easily and efficiently the posture of the object.

Additionally, in this description, the object includes a living body (one portion of a living body), tissue, a perforating device, an opening device, a suture needle, and the like.

The invention according to claim 2 is the gripper according to claim 1, wherein the clipping portion is supported in a rotatable manner on the holding portion, and the posture manipulation portion is a rotation manipulation portion which applies rotation to the clipping portion.

The invention according to claim 6 is the attachment according to claim 5, wherein the clipping portion is supported in a rotatable manner on the holding portion, and the posture manipulation portion is a rotation manipulation portion which applies rotation to the clipping portion.

According to the gripper and the attachment of the present invention, the clipping portion is formed so as to be supported in a rotatable manner on the holding portion, and to be rotated by the rotation manipulation portion. Thereby, it is possible to rotate the object to change the posture of the object easily and efficiently.

The invention according to claim 3 is the gripper according to claim 2, wherein the gripper main body comprises a first arm and a second arm which are connected with each other at a connection portion, and which open and close about the connection portion as a supporting point, wherein opposing surfaces configure the holding portion, the opposing surfaces being formed by open and close of the first arm and the second arm, wherein in the holding portion with respect to the first arm and the second arm, each of a pair of clipping members configuring the clipping portion is connected to the holding portion via the supporting points in a rotatable manner, each of the clipping members is connected to the rotation manipulation portion at a position offset from the supporting point, and applies displacement to the position to which the rotation manipulation portion is connected.

The invention according to claim 7 is the attachment according to claim 6, wherein the gripper main body comprises a first arm and a second arm, which are connected with each other at a connection portion, and are allowed to open and close using the connection portion as a supporting point, and the holding portion is configured by opposing surfaces being formed by open and close of the first arm and the second arm, wherein the attachment further comprises a pair of clipping members being mounted to the first arm and the second arm respectively, and each clipping member being allowed to rotate about a support point relative to each of the first and the second arms, and the rotation manipulation portion is connected with a position of each of the clipping member, the position being offset from the support point, and applies displacement to the position to which the rotation manipulation portion is connected.

According to the gripper and the attachment of the present invention, it is possible to rotate the object to change the posture of object easily and efficiently, by a simple configuration such that the clipping portion, the rotation manipulation portion and the like are added to a conventional gripper device (the gripper main body) such as a forceps type, a Castro Viejo needle holder type, or the like, where the holding portion is formed by open and close of the first and the second arms.

The invention according to claim 4 is a needle holder comprising the gripper according to any one of claims 1 to 3; wherein the object is a suture needle.

The invention according to claim 8 is the attachment according to any one of claims 5 to 7, wherein the gripper main body is a needle holder which clips a suture needle as the object.

According to the needle holder and the attachment of the present invention, the needle holder has: the clipping portion for clipping the suture needle; and the posture manipulation portion, and the posture of the clipping portion is allowed to change. Thereby, it is possible to, by changing efficiently the posture of the suture needle, handle the needle and perform the suture safely and efficiently.

### Effects of Invention

According to the gripper and the attachment of the present invention, since the posture of the object clipped at the clipping portion can be changed by the posture manipulation portion, it is possible to change the posture of the object in a narrow space efficiently.

Additionally, according to the attachment of the present invention, it is possible to configure the gripper easily and efficiently, by adding the attachment to the gripper main body. As a result of that, it is possible to change the posture of the object easily and efficiently.

Further, according to the needle holder of the present invention, since the needle holder has the clipping portion and the rotation manipulation portion, and can change the posture of the clipping portion, it is possible to, by changing efficiently the posture of the suture needle, handle the needle and perform the suture safely and efficiently.

### Brief Description of Drawings

Fig.1 is a diagram showing a schematic configuration of a needle holder according to a first embodiment of the present invention.
Fig.2 is a diagram for explaining the operation of the needle holder of the first embodiment.
Fig.3 is a diagram for explaining the operation of the needle holder of the first embodiment.
Fig.4 is a diagram for explaining the operation of the needle holder of the first embodiment.
Fig.5 is a diagram for explaining the operation of the needle holder of the first embodiment.
Fig.6 is a diagram showing a schematic configuration of a needle holder according to a second embodiment.
Fig.7A is a diagram showing a schematic configuration of a gripper according to a third embodiment.
Fig.7B is a diagram showing a schematic configuration of a gripper according to the third embodiment
Fig.8A is a diagram showing one example of usage mode of the gripper of the third embodiment.
Fig.8B is a diagram showing one example of usage mode of the gripper of the third embodiment.
Fig.9 is a diagram showing a schematic configuration of a needle holder according to a fourth embodiment.
Fig.10 is a diagram showing a schematic configurations of a gripper and an attachment according to a fifth embodiment.
Fig.11 is a diagram showing a schematic configuration of a gripper according to a sixth embodiment.

### Description of Embodiments

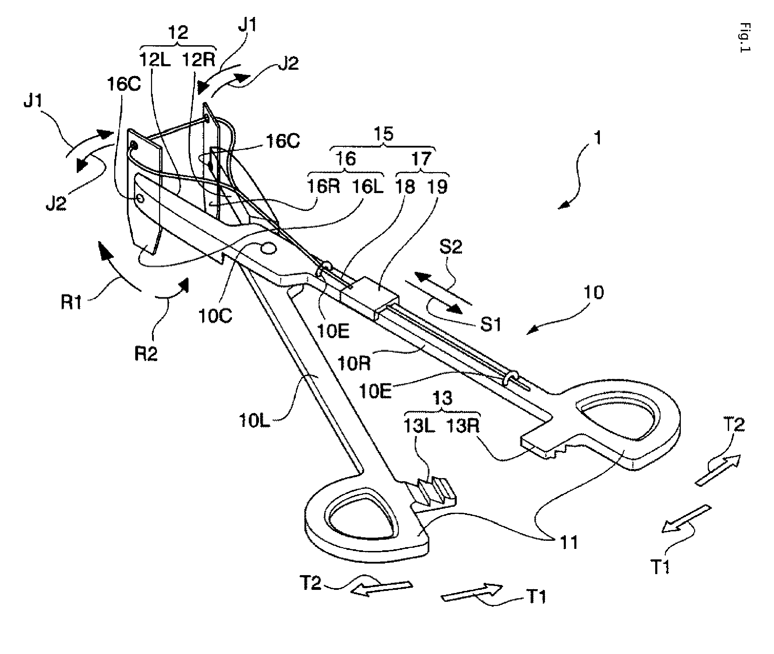

Hereinafter, in reference to Figs.1 to 5, a needle holder (a gripper) according to a first embodiment of the present invention will be described. Fig.1 is a diagram showing a schematic configuration of the needle holder according to the first embodiment. The reference sign 1 indicates the needle holder, and the reference sign 100 indicates a suture needle treated by the needle holder 1.

The needle holder 1 comprises, as shown in Fig.1, a needle holder main body (a gripper main body) 10 and a posture control portion 15. By holding and handling the suture needle 100, for example, it is possible to suture an opening of wound and the like of a living body.

The needle holder main body 10 comprises a first arm 10L, a second arm 10R, and a supporting pin (a connection portion) 10c. Each of the first arm 10L and the second arm 10R has a manipulation portion 11 where a circularity portion is formed, a holding portion 12, and a ratchet portion 13. The first arm 10L and the second arm 10R are connected with each other pivotally via the supporting pin 10c.

Further, the needle holder main body 10 is configured in such a way that the holding portion 12 opens and closes about the supporting pin 10c by manipulating the manipulation portion 11 to make the first arm 10L and the second arm 10R pivot, and by a close manipulation a holding surface (an opposing surface) 12L of the first arm 10L side and a holding surface (an opposing surface) 12R of the second arm 10R side are capable of opposing each other.

When the holding portion 12 is closed in response to the close manipulation of the manipulation portion 11, the ratchet portion 13 gets into an engaged state and a locked state so as to maintain a close state of the needle holder 1. Once the ratchet portion 13 gets into the locked state, the ratchet portion 13 releases the lock state by elastically deforming each of the ratchet pieces 13L and 13R to a side where the ratchet pieces 13L and 13R are separated from each other.

-The posture control portion 15 comprises a clipping portion 16 provided to the holding portion 12, and a rotation manipulation portion (a posture manipulation portion) 17 which is connected with the clipping portion 16 and applies rotation to the clipping portion 16.

The clipping portion 16 has clipping members 16L and 16R which correspond to the holding surfaces 12L and 12R respectively. In the first embodiment, each of the clipping members 16L and 16R is, for example, a board piece which is a rectangle having adjacent corner portions each of which was cut off (chamfered). In the clipping members 16L and 16R, attaching holes are formed near the central portion thereof for attaching to the holding surfaces 12L and 12R respectively. A rotation manipulation hole is formed at a position offset from the attaching hole.

Further, the clipping members 16L and 16R are attached on the holding surfaces 12L and 12R via the supporting members 16C in a rotatable manner respectively.

Each supporting member 16C is configured as a screw which penetrates the attaching hole formed in each of the clipping members 16L and 16R, and which is fixed to each screw hole formed in the holding portion 12. Thereby, the head portions of the screws prevent the clipping members 16L and 16R from removing from the holding surfaces 12L and 12R respectively. In addition, a bearing or something like a bearing may be attached, as necessary.

The rotation manipulation portion 17 comprises a wire 18 and a manipulation button 19. The wire 18 has a circularity portion which is formed so as to go through the rotation manipulation holes of the clipping members 16L and 16R, and a straight line portion. The straight line portion is formed so as to extend to the manipulation portion 11 side through the manipulation button 19. When the wire 18 is made to go back and forth, rotation is applied to the clipping members 16L and 16R.

The manipulation button 19 is, for example, located on the second arm 10R, and the straight line portion of the wire 18 passes through a remove preventing member 10E formed on the second arm 10R, and the straight line portion is allowed to slide in directions shown by arrows S1 and S2 relative to the second arm 10R.

By the above mentioned construction, the needle holder 1 is configured so that, when the manipulation portion 11 is manipulated in a direction of an arrow T1, the clipping portion 16 is closed with the holding portion 12 in a direction of an arrow J1 to clip the suture needle 100, and when the manipulation portion 11 is manipulated in a direction of an arrow T2, the clipping portion 16 is opened in a direction of an arrow J2 with the holding portion 12 to remove the suture needle 100.

Further, the needle holder 1 is configured so as to change the posture of the suture needle 100 in such a way that the clipped suture needle 100 is rotated in a direction of an arrow R1 by manipulating the manipulation button 19 in the direction of the arrow S1, and the clipped suture needle 100 is rotated in a direction of an arrow R2 by manipulating the manipulation button 19 in the direction of the arrow S2,.

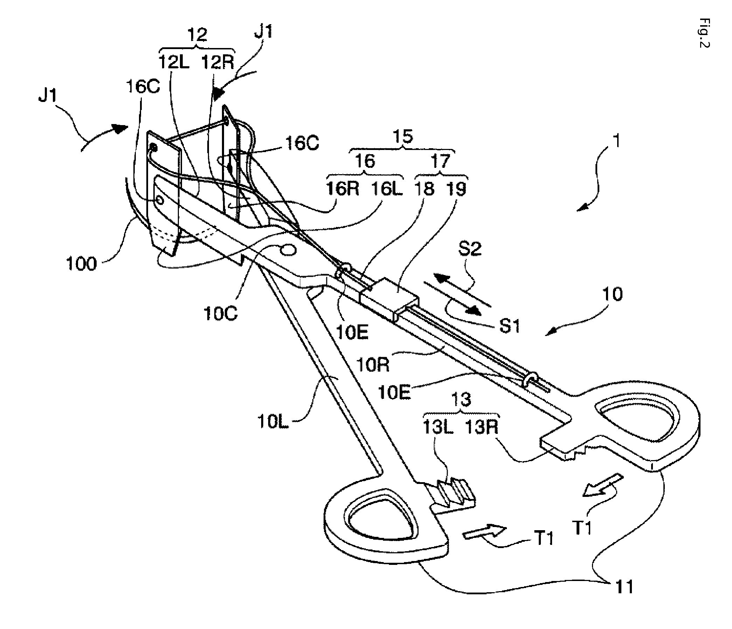

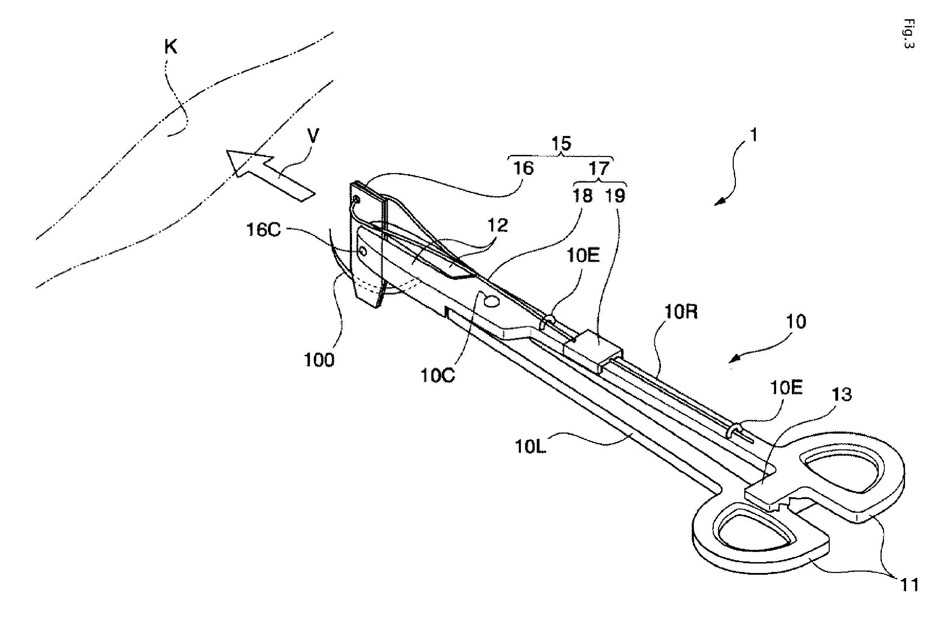

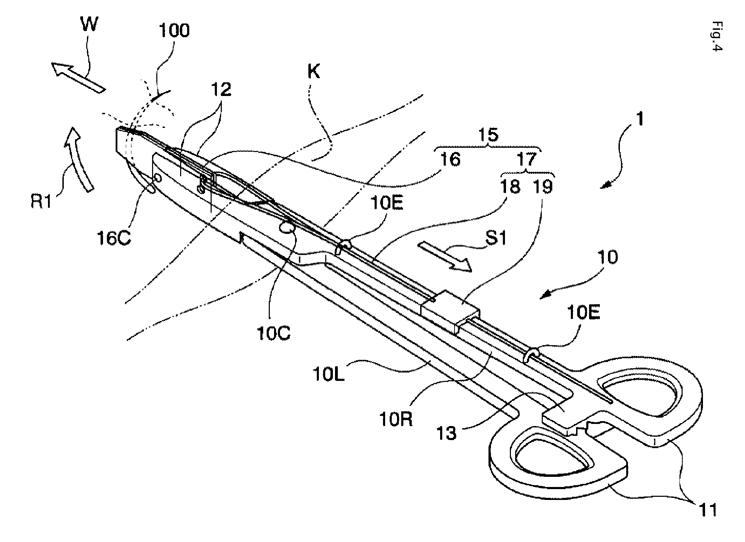

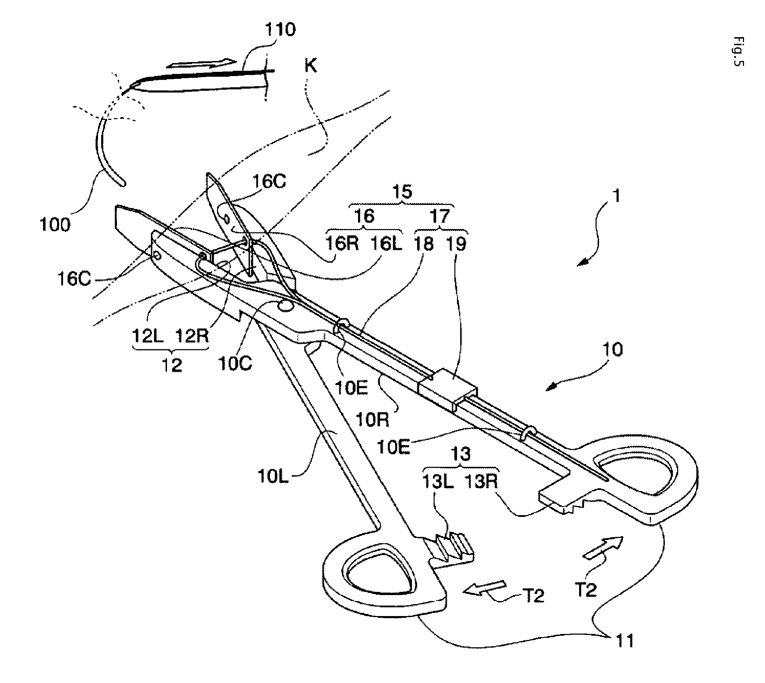

Next, in reference to Figs.2 to 5, the operation of the needle holder 1 according to the first embodiment will be explained.
(1) First, as shown in Fig.2, for example, by manipulating the manipulation button 19 of the rotation manipulation portion 17, an operator makes the holding portion 12 open in a state that the longitudinals of the clipping members 16L and 16R are made to intersect (for example, at a right angle) the longitudinal direction of the needle holder main body 10. After that, by manipulating the manipulation portion 11, the operator makes the clipping members 16L and 16R come close to each other to clip the suture needle 100.
   At this moment, the suture needle 100 is clipped in a direction perpendicular to the longitudinal direction of the suture needle 1.
(2) Next, as shown in Fig.3, the operator makes the suture needle 100 clipped by the clipping portion 16 insert in a direction of an arrow V from an opening K of a living body, the opening K being formed by a surgical operation or the like, and reach a wound (the suturing object) in a narrow space.
(3) Subsequently, as shown in Fig.4, the operator moves the needle holder 1 in a direction of an arrow W and sticks the tip of the suture needle 100 into the living body. After sticking the tip of the suture needle 100 into the living body, the operator makes the manipulation button 19 slide in the direction of the arrow S1 to rotate the suture needle 100 together with the clipping portion 16 in the direction of the arrow R1. Thereby, the operator can handle the suture needle 100 and suture the wound.
(4) Next, as shown in Fig.5, the operator moves the manipulation portion 11 in a direction of an arrow T2 to open the holding portion 12. Thereby the suture needle 100 is removed. Then, the operator pulls out the tip side of the suture needle 100 with, for example, a pincette 110 to suture the living body. And then, the operator manipulates the manipulation portion 11 and the posture control portion 15 of the needle holder 1 to clip the suture needle 100 again, and moves the suture needle 100 to a position to be next sutured.

The operator repeats (3) and (4) until the wound is completely sutured. After suturing the wound completely, the operator takes the needle holder 1 with the suture needle 100 out of the opening K.

In the above (3), explained is a case that the operator makes the suture needle 100 pass in the living body to suture the wound by rotating the suture needle 100 upon the manipulation to the manipulation button 19. However, for example, the following procedure may be employed: after changing the posture of the suture needle 100 upon the manipulation to the manipulation button 19, the operator moves the needle holder 1 in the direction of the arrow W shown in Fig.4; sticks the suture needle 100 into the living body; and handles the suture needle 100.

According to the needle holder 1, the clipping portion 16 is supported on the holding portion 12 in a rotatable manner, and is configured so as to be rotated by the rotation manipulation portion 17. Thereby, it is possible to change the posture of the suture needle 100 easily and efficiently by rotating the suture needle 100.

Further, with respect to the needle holder 1, because the configuration of the needle holder 1 is simple, it is possible to rotate the suture needle 100 easily and efficiently to change the posture of the suture needle 100, and thereby, it is possible to handle the suture needle 100 safely and efficiently.

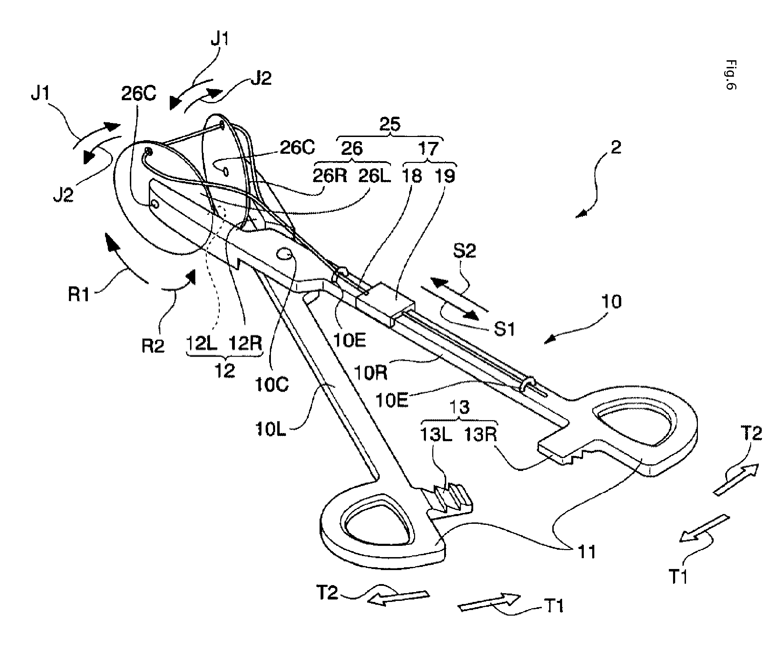

Next, in reference to Fig.6, explained is a needle holder (a gripper) 2 according to a second embodiment of the present invention. The needle holder 2 is different from the needle holder 1 in the following points: the needle holder 2 comprises a posture control portion 25 having a clipping portion 26 formed by disciform clipping members 26L and 26R supported by a supporting member 26C in a rotatable manner instead of the posture control portion 15 which is used in the needle holder 1, the posture control portion 15 having the clipping portion 16 fromed by the rectangular clipping members 16L and 16R supported by the supporting member 16C. The second needle holder 2 is similar to the needle holder 1 of the first embodiment in the other portions. Because of this, with reference to the other portions, identical reference signs are assigned to them respectively, and the explanation thereof will be omitted.

According to the needle holder 2, the clipping members 26L and 26R are formed in the disciform shape. Therefore, a change in the appearance of the needle holder 2 is small even if the rotating angle of the clipping members 26L and 26R is changed. Thereby, it is possible to, while clipping the suture needle 100 at any rotating angle control easily the posture of the suture needle 100 to be handled.

Further, the clipping members 26L and 26R do not have any angle portions due to the disciform shape. Therefore, it is possible to suppress to scratch the periphery of the opening K, a path passing to the narrow space from the opening K, and the living body in the narrow space.

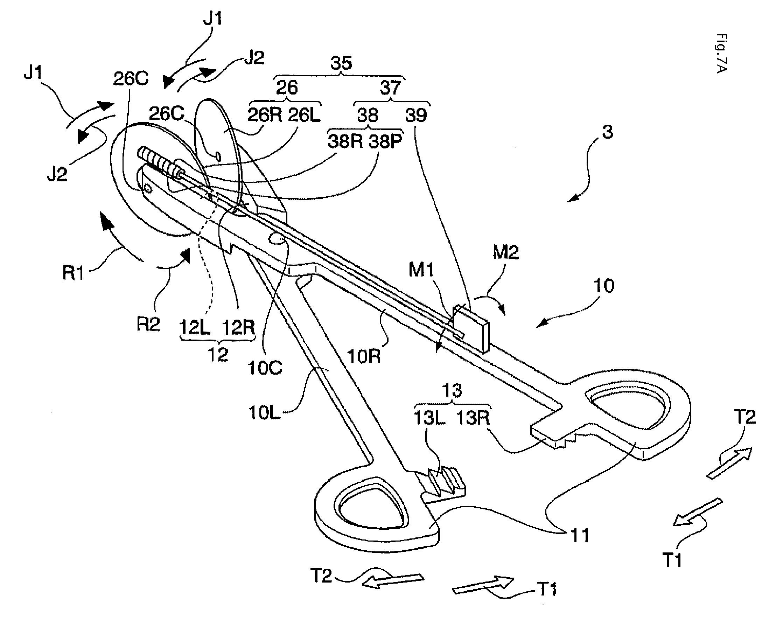

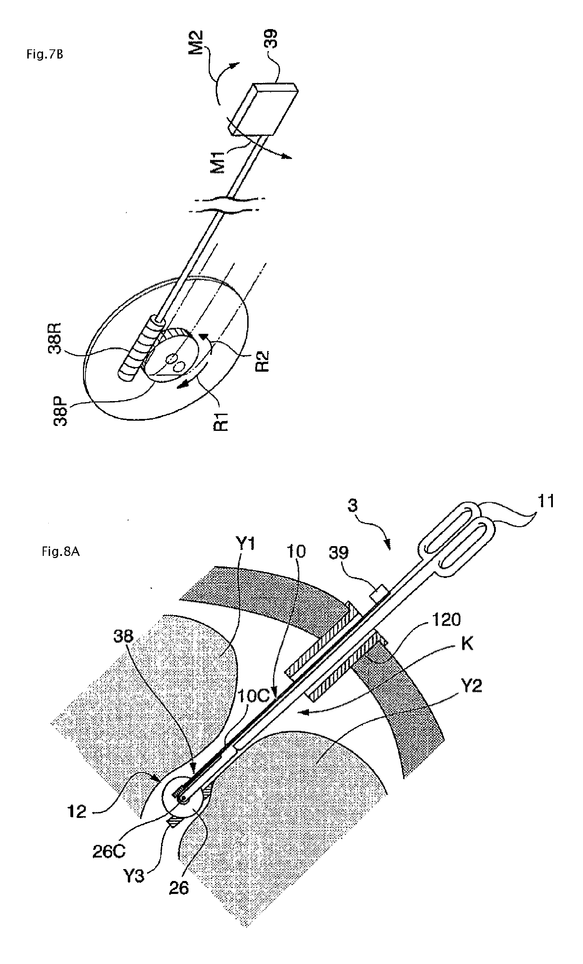

Next, in reference to Figs. 7A and 7B, a gripper 3 according to a third embodiment of the present invention will be explained.
Fig.7A is a diagram showing a schematic configuration the needle holder 3, and Fig.7B is a diagram showing one portion of detailed configuration of a posture control portion.

The gripper 3 is different from the needle holder 2 in the following points: as shown in Fig.7A, the gripper 3 comprises a posture control portion 35 instead of the posture control portion 15; and the object to be clipped is a living body, an instrument for sectioning or the like.

As shown in Figs.7A and 7B, the posture control portion 35 comprises the clipping portion 26 and a rotation manipulation portion 37. The rotation manipulation portion 37 comprises a worm gear mechanism 38 and a manipulation lever 39. The worm gear mechanism 38 is configured by a worm gear (a crossed helical gear) 38R and a wheel (a helical gear) 38P provided on the clipping member 26L coaxially with the supporting member 26C. The manipulation lever 39 is provided to the second arm 10R pivotally by a supporting member not illustrated, and is connected to the worm gear 38R by a connection rod.

According to the configuration mentioned above, the operator can rotate the clipping members 26L and 26R in the direction of the arrow R1 by manipulating the manipulation lever 39 in the direction of the arrow M1, and can rotate the clipping members 26L and 26R in the direction of the arrow R2 by manipulating the manipulation lever 39 in the direction of the arrow M2. Thereby, the gripper 3 is configured so as to rotate the object and control the posture. Since the gripper 3 is similar to the needle holder 1 in the other portions, with respect to the other portions, the identical reference signs are assigned to them, and the explanation thereof is omitted.

According to the gripper 3, since the rotation manipulation portion 37 comprises the worm gear mechanism 38, by the manipulation to the manipulation lever 39, it is possible to change easily and precisely the rotating angle of the clipping portion 26, that is, the posture of the object. Further, even if the operator leaves his/her using fingers from the manipulation lever 39, the positions of the manipulation lever 39 and the clipping portion 26 are held. Therefore, for example, it is possible to hold the positions at an intermediate rotating angle (for example, any direction less than ±90-degree more than 0-degree, in a case that the tip side of the gripper 3 is 0 degree.), and it is possible to perform procedures such as perforation in a state that the operator leaves his/her fingers from the manipulation lever 39.

As a result of that, for example, it is possible to control precisely the posture of the object in the narrow space.

The gripper 3 can be a needle holder. In this case, it is possible to change the posture of the suture needle 100 easily and precisely to face the suture needle 100 in any direction, and possible to provide an efficient handling of the needle and suture in a state that the operator leaves his/her fingers from the manipulation lever 39 to make his/her fingers free. Further, for example, it is possible to improve the safety and the efficiency of the suture procedure by controlling precisely the posture of the suture needle 100 from the opening K to the narrow space and the posture of the suture needle 100 in the narrow space.

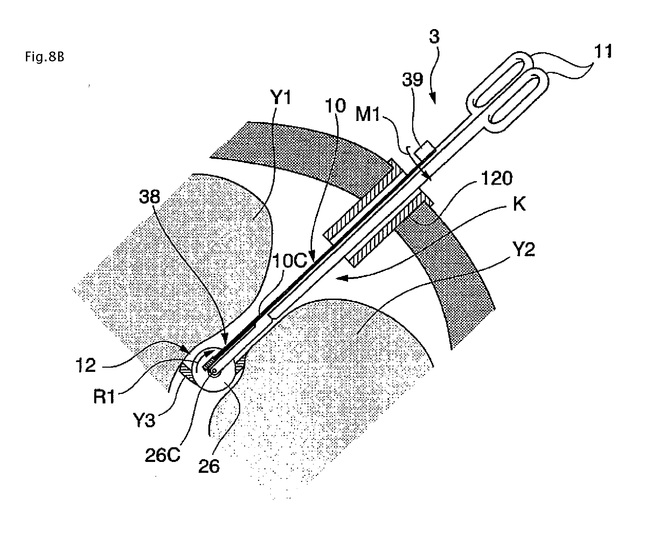

Next, in reference to Figs.8A and 8B, one example of use of the gripper 3 as a third embodiment will be explained.

In Figs.8A and 8B, the reference sign 120 indicates a trocar which is used when the griper 3 or the like are inserted into an abdominal cavity in an abdominal cavity surgery. For example, the figures shows an example that in the abdominal cavity, the direction of a blood vessel Y3 is changed, the blood vessel Y3 existing at the back of living bodies (for example, internal organs or the like) Y1 and Y2.

First, as shown in Fig.8A, the operator grips the blood vessel Y3 with the clipping portion 26.

Next, as shown in Fig.8B, the operator pivots the manipulation button 39 in the direction of the arrow M1 to rotate the clipping position 26 about the supporting pin 26C. Thereby, the direction of the blood vessel Y3 is changed.

By manipulating the gripper 3 as mentioned above, for example, the posture of one portion of the living body or the posture of a tissue can be changed. Thereby, it is possible to perform efficiently a difficult procedure in the narrow space, such as a direction change in a small space.

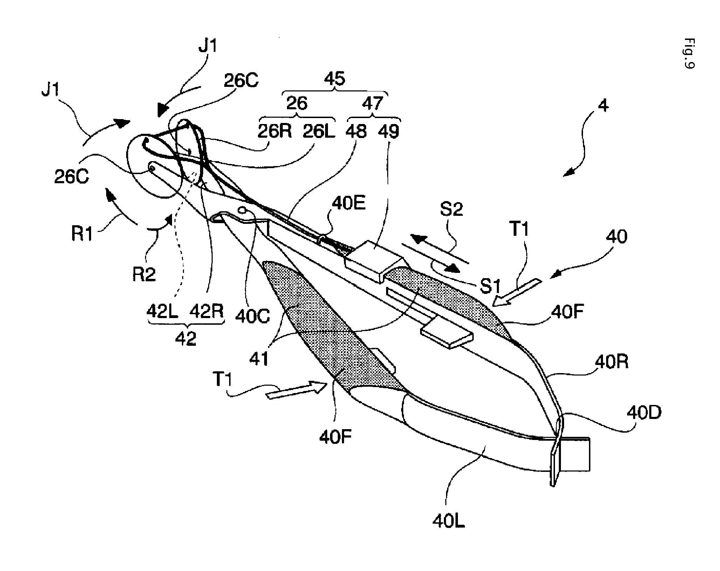

Next, in reference to Fig.9, a needle holder (a gripper) 4 as a fourth embodiment of the present invention will be explained. Fig.9 shows the needle holder 4 where the present invention is applied to a needle holder main body 40 of, for example, the Castro Viejo type. In Fig.9, a posture control portion 45 functions as an attachment which is attachable to the needle holder main body 40.

As shown in Fig.9, the needle holder 4 comprises a needle holder main body 40 and the posture control portion 45. By holding and handling the suture needle 100, for example, the operator can suture an opening of a wound of living body or the like.

The needle holder main body 40 comprises a first arm 40L, a second arm 40R, and a supporting pin (a connection portion) 40C. The first arm 40L and the second arm 40R are connected with each other by a connection portion 40D at hand of the operator. Each of the first arm 40L and the second arm 40r has a manipulation portion 41 and a holding portion 42. The first arm 40L and the second arm 40R are connected with each other via the supporting pin 40C pivotally.

Further, With respect to each of the first arm 40L and the second arm 40R, the portion between the supporting pin 40C and the connection portion 40D is elastically deformable. On the manipulation to the manipulation portion 41, each of the first arm 40L and the second arm 40R pivots about the supporting pin 40C so that the holding portion 42 opens and closes.

When the manipulation portion 41 is pressed in a direction of an arrow T1, each of the first arm 40L and the second arm 40R pivots about the supporting pin 40c, and then, a holding surface (an opposing surface) 42L of the first arm 40L and a holding surface (an opposing surface) 42R of the second arm 40R are closed. When the pressing to the manipulation portion 41 is eased, the first arm 40L and the second arm 40R are restored because of their elasticity so that the holding portion 42 is opened. In addition, on the manipulation portion 41, a gripping portion rubber 40F is formed for easing the manipulation. The attachment may be attached to the needle holder main body 40 by attaching the gripping portion rubber 40F to the first arm 40L and the second arm 40R.

The posture control portion 45 comprises a clipping portion 26 provided to the holding portion 42; and a rotation manipulation portion 47 which is connected to the clipping portion 26 and applies rotation to the clipping portion 26.

The clipping portion 26 comprises the holding members 26L and 26R corresponding to the holding surfaces 42L and 42R respectively. The rotation manipulation portion 47 comprises a wire 48 and a manipulation button 49. The wire 48 passes through an anti-fall member 40E which is formed on the second arm 40R. The clipping portion 26 and the rotation manipulation portion 47 are similar to the case of the needle holder 2 of the second embodiment.

According to the above configuration, the needle holder 4 can clip the suture needle 100 only by the press manipulation. When the manipulation button 49 is manipulated in the direction of the arrow S1, the suture needle 100 clipped can be rotated in the direction of the arrow R1. When the manipulation button 49 is manipulated in the direction of the arrow S2, the suture needle 100 clipped can be rotated in the direction of the arrow R2. Thereby it is possible to change the posture of the suture needle 100.

Further, according to the attachment as which the posture control portion 45 functions, the attachment comprises the clipping portion 26 and the rotation manipulation portion 47. The clipping portion 26 can be attached on the holding portion 42 in a rotatable manner with a screw or the like. Therefore, in the fourth embodiment, by applying the attachment to a conventional needle holder of Castro Viejo type (corresponding to the needle holder main body of the present invention), it is possible to obtain the needle holder 4 easily and also efficiently. As a result of that, it is possible to change the posture of the suture needle 100 easily and also efficiently, and possible to handle the needle safely and also efficiently.

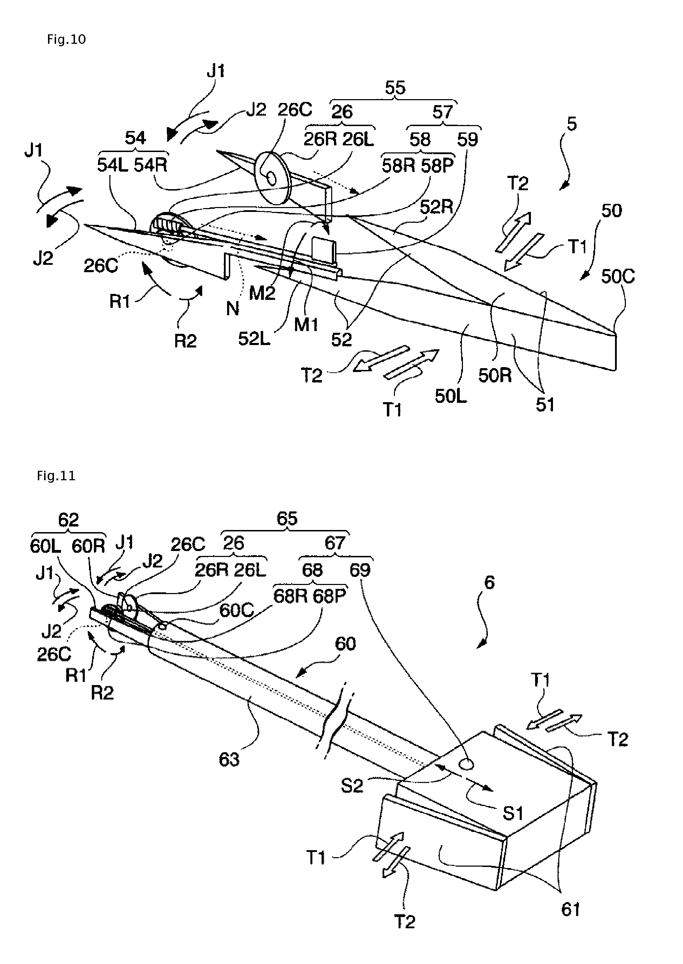

Next, in reference to Fig.10, a gripper 5 according to a fifth embodiment of the present invention will be described.

The gripper 5 comprises, as shown in Fig.10, a pincette (a gripper main body) 50 and a posture control portion 55, and provides posture change of a holding object.

The pincette 50 comprises a first arm 50L, a second arm 50R, a connection portion 50C, a manipulation portion 51, and a holding portion 52. The first arm 50L and the second arm 50R are flexible, and because of elastic deformation of the connection portion 50C, the first arm 50L and the second arm 50R open and close so that holding surfaces (opposing surfaces) 52L and 52R of the holding portion 52 faces each other.

Namely, when the operator grips the manipulation portion 51 of the first and second arms 50R and 50L and presses them in the direction of the arrow T1, the connection portion 50C is inflected so that the first and second arms 50L and 50R are closed in the direction of the arrow J1. When the operator stops pressing them, the elastic deformation of the connection portion 50c is released, and the first and second arms 50L and 50R and the holding portion 52 moves in directions of the arrows T2 and J2 respectively.

In this embodiment, in a case that the pincette 50 is considered as the gripper 5, an attachment is configured by the posture control portion 55 and sucks 54 (54L and 54R). The posture control portion 55 is provided to, for example, the suck 54L. The attachment is configured so that the suck 54 is moved in the direction of an arrow N to be attached to the pincette 50.

The posture control portion 55 comprises the clipping portion 26 provided to the holding portion 52 and a rotation manipulation portion 57 which is connected with the clipping portion 26 to apply rotation to the clipping portion 26. The clipping portion 26 comprises the clipping members 26L and 26R and the rotation manipulation portion 57. The clipping members 26L and 26R correspond to the holding surfaces 52L and 52R respectively, and each of the clipping members 26L and 26R is allowed to rotate by the supporting member 26C. The clipping portion 26 is configured similarly to the second embodiment.

The rotation manipulation portion 57 comprises a worm gear mechanism 58 and a manipulation lever 59. The worm gear mechanism 58 has a worm gear (a crossed helical gear) 58R and a wheel (a helical gear) 58P formed on the clipping member 26L coaxially with the supporting member 26C. The manipulation lever 59 is provided to the second arm 10R by a supporting member not illustrated, and connected with the worm gear 58R by a connection rod.

According to the gripper 5, the operator rotates the clipping members 26L and 26R in the direction of the arrow R1 by manipulating the manipulation lever 59 in the direction of the arrow M1, or rotates the clipping members 26L and 26R in the direction of the arrow R2 by manipulating the manipulation lever 59 in the direction of the arrow M2. Thereby, it is possible to control the rotation and posture of the object.

Next, in reference to Fig.11, a gripper 6 according to a sixth embodiment of the present invention will be described.

The gripper 6 comprises, as shown in Fig.11, a gripper main body 60 and a posture control portion 65. The gripper 6 is capable of gripping an object such as a living body during, for example, an abdominal cavity surgery.

The gripper main body 60 has two manipulation levers (a manipulation portion) 61 extending from a manipulation portion main body (a chassis) and a holding portion 62. The holding portion 62 has a first arm 60L, a second arm 60R and a connection portion 60c. The first and second arms 60L and 60R are connected with each other pivotally via the connection portion 60C.

The manipulation levers 61 are connected with the holding portion 62 via a transmission mechanism not illustrated. The transmission mechanism is housed in a rod 63 and the like which is appropriate for manipulating the holding portion 62 existing in the abdominal cavity from the outside of the living body. When the operator presses the manipulation levers 61 in the direction of the arrow T1, the first and second arms 60L and 60R close in the direction of the arrow J1. When the operator stops pressing them, the manipulation levers 61 returns in the direction of the arrow T2 so that the first and second arms 60L and 60R open in the direction of the arrow J2.

The posture control portion 65 comprises the clipping portion 26 and a rotation manipulation portion 67 which is connected with the clipping portion 26 and applies rotation to the clipping portion 26.

The clipping portion 26 comprises the clipping members 26L and 26R which correspond to the first arm 60L and the second arm 60R respectively, and are allowed to rotate by the supporting members 26C. The rotation manipulation portion 67 comprises a worm gear mechanism 68 having a worm gear (a screw gear) 68R and a wheel (a helical gear) 68P formed on the clipping member 26L coaxially with the supporting member 26C; and a manipulation button 69. The worm gear 68R is, for example, connected with a motor (not illustrated) via a transmission mechanism, which is a wire or the like arranged in the rod 63, and is rotated due to a manipulation to the manipulation button 69. The clipping portion 26 is configured similarly to the second embodiment.

According to the above configuration, the gripper 6 makes the operator press the manipulation button 61 to grip the object by the clipping portion 26. For example, by manipulating the manipulation button 69 in the directions of the arrows S1 and S2, a clipped object is rotated in the directions of the arrows R1 and R2. Thereby, it is possible to change the posture of the clipped object.

The present invention is not limited to the above mentioned embodiments, and includes variable matters within the scope of the invention.

For example, the needle holders are explained as the embodiments 1, 2, and 4, and the grippers are explained as the embodiments 3, 5, and 6. However, as mentioned above the needle holder is one of embodiments for use of the gripper, and it is possible to select arbitrarily any embodiment as the gripper.

In addition, in the above mentioned embodiment, in the fourth and fifth embodiments, explained is the attachment for the needle holder 4 or the gripper 5. However, for example, the attachment may be formed based on the clipping portion and the rotation manipulation portion relating to neither the needle holder 4 nor the gripper 5.

It is not necessary that the needle holder 4 or the gripper 5 is completed after the attachment is attached thereto, and the needle holder 4 or the gripper 5 may be distributed as a complete product. In this case, it is possible to determine arbitrarily whether a supporting member is included in the structure of the attachment.

Further, in the above embodiment, explained are the cases that the present invention is applied to a forceps type, a Castro Viejo needle holder type, a pincette, and a gripper main body for abdominal cavity surgery. However, the present invention may be applied to a gripper main body (including a needle holder main body) other than the above mentioned ones.

When the attachment is attached to the gripper main body or the like, the attachment may be attached so as to house one portion or all of the gripper main body or the like, or may be attached to engage with somewhere in the gripper main body or the like. For attaching the attachment, well-known means, such as, a screw, weld, adhesion and the like can be applied.

Further, in the above embodiments, explained is the case that the clipping member 16 is the board piece which is a rectangular having adjacent corners thereof which were cut (chamfered), and the clipping member 26 has the disciform shape. However, for example, the board piece formed in a shape other than the above mentioned shape may be applied. Or, for reliably clipping a suture needle, a single groove or plural grooves may be formed on the clipping member, or plural convex portions may be located dispersedly on the clipping member.

Further, in the above embodiments, explained is the case that the clipping portions 16 and 26 are supported by the supporting members 16c and 26c so as to rotate at ±90 degrees. However, the clipping portions 16 and 26 may be constructed so that the rotating angle of the clipping portions 16 and 26 is determined arbitrarily. Or, the clipping portions 16 and 26 may be constructed so that the angle of the rotation is controlled, or, so that the rotation is stopped at a desired angle.

Further, in the above embodiments, explained are the cases that the posture control portions 15, 25, 35, 45, 55 and 65 support the clipping portions 16 and 26 by the supporting members 16c and 26c which are rotatable about one axis. However, for example, the posture control portion may be constructed so that the postures of the clipping portion is controlled about plural axes or other situation by making a supporting member rotatable about plural axes (for example, X axis and y axis), a supporting member of the polar coordinate system, or a supporting member including the slide mechanism or the twisted construction, support the clipping portion.

Further, in the above embodiments, explained are the cases that the rotation manipulation portions 17 and 47 rotate the clipping portions 16 and 46 using the wires 18 and 48, and the cases that the rotation manipulation portions 37, 57, 67 rotate the clipping members 26L and 26R using the worm gear mechanisms (the worm gear 38R and the wheel 38P, the worm gear 58R and the wheel 58P, and the worm gear 68R and the wheel 68P). However, for example, the postures of the clipping portions 16 and 26 may be controlled by using a gear, a rack and pinion, a cam, an actuator (for example, a memory metal, a piezoelectric element, a motor or the like), and a spring, instead of the wires 18 and 48 and the worm gear mechanisms.

### Industrial applicability

According to the gripper, the needle holder, and the attachment of the present invention, it is possible to grip an object efficiently. Thereby, the preset invention has the industrial applicability.

### Explanation of References

- Y3: Blood vessel (the object)
- 1, 2, 4: A needle holder (A gripper)
- 3, 5, 6: A gripper
- 10, 40, 50, 60: A gripper main body
- 10C, 40C, 60C: A supporting pin (A supporting point)
- 10L, 40L, 50L, 60L: A first arm
- 10R, 40R, 50R, 60R: A second arm
- 11, 41, 51, 61: A manipulation portion
- 12, 42, 52, 62: A holding portion
- 16, 26: A clipping portion
- 16L, 16R, 26L, 26R: A clipping member
- 16C, 26C, 46C: A supporting member (A supporting point)
- 15, 25, 35, 45, 55, 65: A posture control portion
- 17, 37, 47, 57, 67: A rotation manipulation portion
- 100: A suture needle

## Claims

1. A gripper for gripping an object, comprising:
a gripper main body which has a manipulation portion and a holding portion, where the holding portion opens and closes by manipulation to the manipulation portion, and the holding portion closes to hold the object; and
a posture control portion which controls a posture of the object held by the holding portion, wherein
the posture control portion comprises:
a clipping portion being provided to the holding portion, which clips the object and changes a posture of the clipping portion relative to the holding portion in a state of clipping the object; and
a posture manipulation portion being connected with the clipping portion, which manipulates the posture of the clipping portion.

2. The gripper according to claim 1, wherein
the clipping portion is supported in a rotatable manner on the holding portion, and
the posture manipulation portion is a rotation manipulation portion which applies rotation to the clipping portion.

3. The gripper according to claim 2, wherein
the gripper main body comprises a first arm and a second arm which are connected with each other at a connection portion, and which open and close about the connection portion as a supporting point, wherein opposing surfaces configure the holding portion, the opposing surfaces being formed by open and close of the first arm and the second arm, wherein
in the holding portion with respect to the first arm and the second arm, each of a pair of clipping members configuring the clipping portion is connected to the holding portion via the supporting points in a rotatable manner, each of the clipping members is connected to the rotation manipulation portion at a position offset from the supporting point, and applies displacement to the position to which the rotation manipulation portion is connected.

4. A needle holder comprising the gripper according to any one of claims 1 to 3; wherein
the object is a suture needle.

5. An attachment being applied to a gripper main body having a manipulation portion and a holding portion, the holding portion being allowed to open and close by manipulation to the manipulation portion, and closing to hold an object, wherein
the attachment has a posture control portion which controls a posture of the object held by the holding portion, and
the posture control portion has:
a clipping portion which is provided to the holding portion, and which clips the object and is allowed to change a posture of the clipping portion relative to the holding portion while clipping the object; and
a posture manipulation portion which is connected with the clipping portion, and operates the posture of the clipping portion.

6. The attachment according to claim 5, wherein
the clipping portion is supported in a rotatable manner on the holding portion, and
the posture manipulation portion is a rotation manipulation portion which applies rotation to the clipping portion.

7. The attachment according to claim 6, wherein
the gripper main body comprises a first arm and a second arm, which are connected with each other at a connection portion, and are allowed to open and close using the connection portion as a supporting point, and the holding portion is configured by opposing surfaces being formed by open and close of the first arm and the second arm, wherein
the attachment further comprises a pair of clipping members being mounted to the first arm and the second arm respectively, and each clipping member being allowed to rotate about a support point relative to each of the first and the second arms, and
the rotation manipulation portion is connected with a position of each of the clipping member, the position being offset from the support point, and applies displacement to the position to which the rotation manipulation portion is connected.

8. The attachment according to any one of claims 5 to 7, wherein the gripper main body is a needle holder which clips a suture needle as the object.
